(19) **Europäisches Patentamt**

**European Patent Office**

**Office européen des brevets**

(11) **EP 1 312 380 B1**

(12) **EUROPEAN PATENT SPECIFICATION**

(45) Date of publication and mention
of the grant of the patent:
**03.05.2006 Bulletin 2006/18**

(51) Int Cl.:
*A61K 47/04* (2006.01)   *A61K 47/18* (2006.01)
*A61K 47/32* (2006.01)   *A61K 47/38* (2006.01)
*A61K 9/08* (2006.01)   *A61P 27/02* (2006.01)
*A01N 59/14* (2006.01)   *A61L 12/14* (2006.01)
*A01N 59/14* (2006.01)   *A01N 43/36* (2006.01)
*A01N 43/16* (2006.01)   *A01N 37/44* (2006.01)

(21) Application number: **01938626.7**

(22) Date of filing: **13.06.2001**

(86) International application number:
**PCT/JP2001/005004**

(87) International publication number:
**WO 2001/097852 (27.12.2001 Gazette 2001/52)**

(54) **ASEPTICS**

ASEPTISCHE MITTEL

AGENTS ASEPTIQUES

(84) Designated Contracting States:
**AT BE CH CY DE DK ES FI FR GB GR IE IT LI LU
MC NL PT SE TR**

(30) Priority: **19.06.2000 JP 2000182624**

(43) Date of publication of application:
**21.05.2003 Bulletin 2003/21**

(73) Proprietor: **SANTEN PHARMACEUTICAL CO.,
LTD.**
**Higashiyodogawa-ku,
Osaka-shi,
Osaka 533-8651 (JP)**

(72) Inventors:
• **MORISHIMA, Kenji**
  **Higashiyodogawa**
  **Osaka-shi**
  **Osaka 533-8651 (JP)**
• **HATANO, Norihisa**
  **Higashiyodogawa**
  **Osaka-shi**
  **Osaka 533-8651 (JP)**

(74) Representative: **Peaucelle, Chantal et al**
**Cabinet Armengaud Aîné**
**3, Avenue Bugeaud**
**75116 Paris (FR)**

(56) References cited:
**EP-A- 0 358 447**        **EP-A2- 0 213 514**
**WO-A-93/18764**        **JP-A- 1 294 620**
**US-A- 5 591 426**        **US-A- 5 663 170**

• **DATABASE EPODOC [Online] EUROPEAN
PATENT OFFICE, THE HAGUE, NL; XP002242055
& CN 1 185 953 A (LIU WEIZHONG) 1 July 1998
(1998-07-01)**

**Description**

The present invention relates to the use of a combination of boric acid, ethylenediaminetetraacetic acid and polyvinyl pyrrolidone in the manufacture of a preservative, and preservatives further containing cellulosic polymers combined therewith. These preservatives can be suitably used for aqueous liquid preparations such as ophthalmic solutions and solutions for contact lenses.

Background Art

Benzalkonium chloride, benzethonium chloride, sorbic acid and the like have been used as preservatives for ophthalmic solutions and solutions for contact lenses.

Though benzalkonium chloride and benzethonium chloride have excellent preservation effects, they are likely to cause corneal disorders depending on concentrations, and the concentrations to be used are limited. Further, these compounds are apt to be adsorbed to contact lenses and plastic containers.

Though sorbic acid, which is widely used as a preservative for ophthalmic solution for contact lenses, exhibits few side-effects and is hardly adsorbed to contact lenses and plastic containers, it has the problem that the preservation effect is weak.

On the other hand, components of the preservatives which can be used for pharmaceuticals such as ophthalmic solutions are limited

EP 0 358 447 discloses compositions suitable for the treatment of contact lenses. In *table 7*, an exemple of aqueous colution is given which comprises 2% polyvinyl pyrrolidone, 0.6 % boric acid, 0.1 % trisodium EDTA and 0.32 % hydroxyethyl cellulose.

US 5 663 170 discloses ophtalmic artificial tear solutions. In exemple 1, an aqueous solution is prepared, including polyvinylpyrrolidone (0.1 %), hydroxypropyl cellulose (0.5 %); edetate disodium (0.03 %), boric acid/sodium borate (resp. 0.3 %) and 0.035 %.

CN 1 186 953 discloses eye drops containing among other ingredients boric acid (0-2.5), borax (0-1), PVP (0-9.5), hydroxypropyl cellulose (0-9), hydroxyethycellulose (0-3.5) and EDTA-NA$_2$ (0-1.2).

WO 9318764 discloses ophtalmic solutions containing boric acid, preferentially 2.8 %, disodium edetate, preferentially 0.18 <ù and PVP, preferentially 2.0 %.

US 5 591 426 discloses compositions suitable for being used in eye drops. An example of such a composition is given in example 18, which is an aqueous solution comprising, among other ingredients, 2% PVP, 0.1% EDTA-NA$_2$, 2 % boric acid and 1 % borax.

Disclosure of the Invention

The present inventors studied precisely to find the use of a combination in the manufacture of preservatives which exhibit preservation effects by using a combination of components already used for aqueous preparations such as ophthalmic solutions with each other. Boric acid and/or borax is widely used as a buffer, and ethylenediaminetetraacetic acid or a salt thereof is used as a stabilizer in ophthalmic solutions. Focusing attention on the fact that these compounds have preservation effects, although weak, the present inventors studied to improve the preservation effects. As a result, the present inventors found that the preservation effect is remarkably increased by combining (C) polyvinyl pyrrolidone, which is widely used as a thickener, to (A) boric acid and/or borax and (B) ethylenediaminetetraacetic acid and completed the present invention. It was also found that the preservation effect is further enhanced by adding cellulosic polymers, which are also widely used as thickeners, to the combination.

The use of the present invention is the combination, in the manufacture of preservative, of three essential components. The first component is boric acid and/or borax. The amount of boric acid and/or borax is 0.05 to 3.0% by weight, preferably 0.5 to 2.0% by weight. When the amount of the first component is too small, a sufficient preservation effect cannot be obtained. A too large amount is not preferable in terms of safety for eyes.

The second component in the use according to the present invention is ethylenediaminetetraacetic acid or a salt thereof. A tetrasodium salt or a disodium salt (disodium edetate) can be suitably used as the salt. The amount of ethylenediaminetetraacetic acid or the salt thereof is 0.01 to 0.3% by weight, preferably 0.05 to 0.2% by weight. When the amount of the second component is too small, sufficient stability and preservation effect cannot be obtained. A too large amount is not preferable in terms of safety for eyes.

The third component in the use according to the present invention is polyvinyl pyrrolidone (PVP). "PVP K-25" (average molecular weight: 25,000), "PVP K-30" (average molecular weight: 40,000) and "PVP K-90" (average molecular weight: 360,000), for example, can be used. The amount of PVP is 0.02 to 4.0% by weight, preferably 0.1 to 2.0% by weight. When the amount of the third component is too small, a sufficient preservation effect cannot be obtained. A too large amount is not preferable since unpleasant stickiness is felt.

The preservation effect is further enhanced by adding the cellulosic polymer to the above-mentioned three components in the use according to the present invention. Examples of the cellulosic polymer are hydroxypropylmethyl cellulose, hydroxypropyl cellulose, methyl cellulose and hydroxyethyl cellulose. Hydroxypropylmethyl cellulose is particularly preferable. An amount of the cellulosic polymer is preferably 0.01 to 0.5% by weight, more preferably 0.05 to 0.3% by weight, but not limited to the ranges.

Since the preservatives manufactured in the use according to the present invention are safe for the human body and hardly adsorbed to contact lenses and plastic containers, these are suitably used for aqueous liquid preparations such as ophthalmic solutions and solutions for contact lenses. It is possible to add appropriately further additive components such as an isotonic agent, a pH adjustor, a solubilizer and another preservative to the above-mentioned components in order to prepare these aqueous liquid preparations.

Drugs to which these aqueous liquid preparations can be applied are not particularly limited and are exemplified by various vitamins (vitamin B2, vitamin B6, vitamin B12, vitamin E, panthenol and the like), decongestants (tetrahydrozoline hydrochloride, naphazoline hydrochloride and the like), anti-inflammatories (disodium glycyrrhizinate, ε-aminocapronic acid and the like), antihistamines (chlorpheniramine maleate, diphenhydramine hydrochloride and the like), antiallergics (sodium cromoglicate and the like), antimicrobials (sulfamethoxazole and the like), amino acids (potassium L-aspartate, aminoethylsulfonic acid, sodium chondroitin sulfate and the like), sodium hyaluronate, neostigmine methylsulfate and the like.

Examples of the isotonic agent are glycerin, propylene glycol, sodium chloride, potassium chloride, sorbitol and mannitol.

Examples of the pH adjustor are hydrochloric acid, citric acid, phosphoric acid, acetic acid, sodium hydroxide, potassium hydroxide, sodium carbonate and sodium hydrogencarbonate.

Examples of the solubilizer are Polysorbate 80, polyoxyethylene hydrogenated castor oil 60 and macrogol 4000.

The preservative manufactured in the use according to the present invention can be combined with a widely-used preservative such as sorbic acid, potassium sorbate, benzalkonium chloride, benzethonium chloride, methyl p-hydroxybenzoate, propyl p-hydroxybenzoate and chlorobutanol. Since the preservative manufactured in the use according to the present invention can complement a preservation effect of the widely-used preservative owing to the combination, the preservative manufactured in the use according to the present invention has also an advantageous effect on decreasing an amount of the widely-used preservative remarkably.

When the liquid preparations of the present invention are used as ophthalmic solutions, it is desirable to adjust pH to about 7.0, and it is desirable to adjust an osmotic pressure ratio to about 1.0.

The present invention is described in detail by giving Examples below, but these Examples do not limit the scope of the present invention.

Best Mode for Carrying out the Invention

Preservation effect tests were carried out according to the following method in order to study preservation effects of the preservative manufactured in the use according to the present invention.

Preservation effect tests

In Examples 1 to 4 and Comparative Examples 1 to 3, formulation ingredients shown in Table 1 were added to distilled water by the conventional method to prepare liquid preparations. Sodium chloride as an isotonic agent was added to each liquid preparation to adjust osmotic pressure to 1.0, and further sodium hydroxide was optionally added to the liquid preparation to adjust pH to 7.0. Preservation effect tests were carried out according to the preservation effect test method of 13th revised Japanese Pharmacopoeia. Staphyrococcus aureus (*S.aureus*) was used as test bacteria, and survival rates of the bacteria were calculated according to the following equation. The obtained values are shown in Table 1.

Survival rate (%) = [(Bacteria number after two weeks) (Initial bacteria number)]×100

Table 1

| Formulation ingredient (% by weight) | Examples | | | | Comparative Examples | | |
|---|---|---|---|---|---|---|---|
| | 1 | 2 | 3 | 4 | 1 | 2 | 3 |
| Boric acid | 1.5% | 1.39% | 1.5% | 1.39% | 1.5% | | |
| Borax | | 0.18% | | 0.18% | | | |
| Na edetate | 0.1% | 0.1% | 0.1% | 0.1% | 0.1% | | |
| PVP[*1] | 1.0% | 0.2% | 1.0% | 1.0% | | 1.0% | |
| HPMC[*2] | | | 0.2% | 0.3% | | | 0.1% |
| Survival rate (%) | 0.50 | 0.59 | 0.19 | 0.03 | 1.7 | 6.2 | 7.9 |
| [*1]PVP: Polyvinyl pyrrolidone [*2]HPMC: Hydroxypropylmethyl cellulose | | | | | | | |

Table 1 explicitly shows that preservation effects are improved remarkably in Examples 1 and 2 of the present invention compared with those in Comparative Examples 1 to 3. Preservation effects are improved in Examples 3 and 4 wherein HPMC is further added compared with those in Examples 1 and 2. Thus, the preservation effects of the preservatives manufactured in the use according to the present invention containing (A) boric acid and/or borax, (B) ethylenediaminetetraacetic acid or a salt thereof and further (C) polyvinyl pyrrolidone in the amounts previously mentioned are improved The preservation effects in the use according to the present invention can be more improved by further adding the cellulosic polymer to the preservatives. Since the preservatives manufactured in the use according to the present invention are prepared to improve the preservation effects of the liquid preparations by combining the compounds widely used as a buffer, a stabilizer and a thickener respectively, the preservatives are safe for the human body and appropriate to pharmaceutical use. The preservatives manufactured in the use according to the present invention can also be combined with a widely-used preservative such as benzalkonium chloride or sorbic acid, and an amount of the widely-used preservative can be reduced owing to the combination.

Industrial Applicability

The use according to the present invention provides preservatives comprising a combination of boric acid, ethylenediaminetetraacetic acid and polyvinyl pyrrolidonein the amounts previously mentioned, and preservatives further containing cellulosic polymers combined therewith. The preservatives can be suitably used for aqueous liquid preparations such as ophthalmic solutions and solutions for contact lenses.

**Claims**

1. Use a combination of (A) boric acid and/or borax in an amount of 0.05 to 0.3 % by weight, (B) ethylenediaminetetraacetic acid or a salt thereof in an amount of 0.01 to 0.3 % by weight and (C) polyvinyl pyrrolidone in an amount of 0.02 to 4.0 % by weight in the manufacture of a preservative

2. The use acording to claim 1, wherein (D) a cellulosic polymer is further combined therewith.

3. The use according to claim 1 or 2 for preparing an aqueous liquid preparation containing the preservative.

4. The use according to claim 3, wherein a dosage form is an ophthalmic solution.

5. The use according to claim 3, which further comprises (D) a cellulosic polymer in an amount of 0.01 to 0.5 % by weight.

6. The use according to claim 2 or 5, wherein the cellulosic polymer is hydroxypropylmethyl cellulose, hydroxypropyl cellulose, methyl cellulose or hydroxyethyl cellulose.

**Patentansprüche**

1. Verwendung einer Kombination aus (A) Borsäure und/oder Borax mit einem Anteil von 0,05 bis 0.3 Gew.-%, (B) Ethylendiamintetraessigsäure oder einem Salz davon mit einem Anteil von 0,01 bis 0,3 Gew.-% und (C) Polyvinylpyrrolidon mit einem Anteil von 0,02 bis 4,0 Gew.-% zur Herstellung eines Konservierungsmittels.

2. Verwendung nach Anspruch 1, wobei außerdem (D) ein Cellulosepolymer damit kombiniert wird.

3. Verwendung nach Anspruch 1 oder 2 zur Herstellung eines das Konservierungsmittel enthaltenden wäßrigen flüssigen Präparats.

4. Verwendung nach Anspruch 3, wobei die Darreichungsform Augentropfen sind.

5. Verwendung nach Anspruch 3, die ferner (D) eine Cellulosepolymer mit einem Anteil von 0,01 bis 0,5 Gew.-% umfasst.

6. Verwendung nach Anspruch 2 oder 5, wobei das Cellulosepolymer Hydroxypropylmethylcellulose, Hydroxypropylcellulose, Methylcellulose oder Hydroxyethylcellulose ist.


**Revendications**

1. Utilisation d'une combinaison (A) d'acide borique et/ou de borax à raison de 0,05 à 0,3 % en poids, (B) d'acide éthylènediaminetetraacétique ou d'un sel de cetacide à raison de 0,01 à 3 % en poids et (C) de polyvinyle pyrrolidone à raison de 0,02 à 4 % en poids pour la fabrication d'un conservateur.

2. Utilisation selon la revendication 1, **caractérisée en ce qu'**un polymère cellulosique (D) est en outre combiné avec ladite combinaison

3. Utilisation selon la revendication 1 ou 2, pour préparer une préparation liquide aqueuse contenant le conservateur.

4. Utilisation selon la revendication 3, **caractérisée en ce qu'**une forme de dosage est une solution ophtalmique.

5. Utilisation selon la revendication 3, **caractérisée en ce qu'**elle comprend en outre un polymère cellulosique (D) à raison de 0,01 à 0,5 % en poids.

6. Utilisation selon la revendication 2 ou 5, **caractérisée en ce que** le polymère cellulosique est de l'hydroxypropylméthyle cellulose, hydroxypropyle cellulose, méthyle cellulose ou hydroxyéthyle